# EUROPÄISCHE PATENTANMELDUNG

(11) **EP 1 671 956 A1**
(43) Veröffentlichungstag der Anmeldung: **21.06.2006**
(21) Anmeldenummer: 05026431.6
(22) Anmeldetag: 03.12.2005
(51) Int. Cl.: C07D 263/14, C08K 5/353, C09C 3/08, C09D 5/03

(54) **Oxazolinmischung und ihre Verwendung als Verarbeitungshilfsmittel für Kunststoffe und Pigmente**

(30) Priorität: 18.12.2004 DE 102004061037
(71) Anmelder: Clariant Produkte (Deutschland) GmbH, 65929 Frankfurt am Main (DE)
(72) Erfinder: Heinrichs, Franz-Leo, Dr., 86456 Gablingen (DE)

(57) **Zusammenfassung**

Die Erfindung betrifft eine Oxazolinmischung enthaltend Oxazoline der Formeln I bis VI 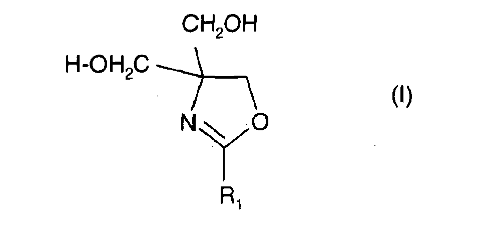 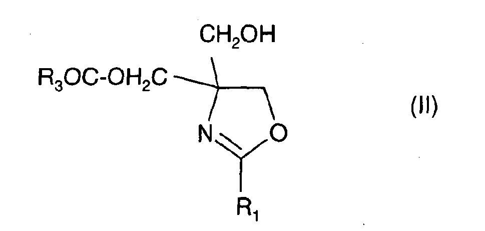 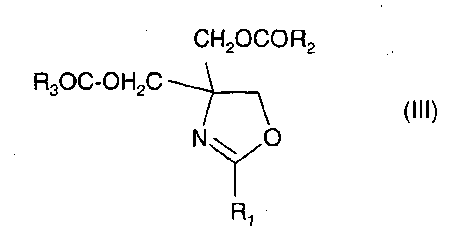 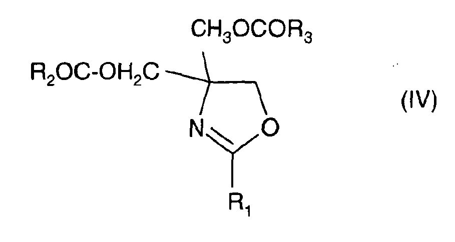 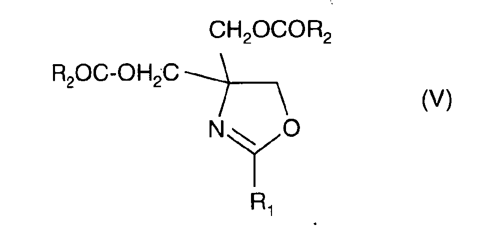 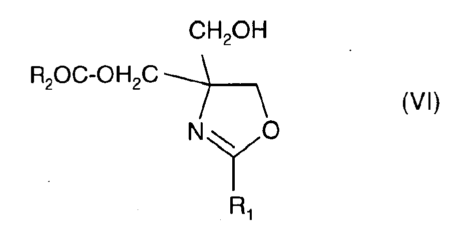 in Mengen von I = 0 bis 10 Mol-%, II = 1 bis 90 Mol-%, III = 1 bis 98 Mol-%, IV = 1 bis 98 Mol-%, V = 0 bis 50 Mol-%, VI = 0 bis 50 Mol-%, wobei R₁ für den Alkylrest einer Fettsäure oder einer Hydroxyfettsäure, und R₂ für den Alkylrest einer Fettsäure, Hydroxyfettsäure und/oder Montanwachssäure und R₃ für den Alkylrest der Montanwachssäure steht, ein Verfahren zu deren Herstellung und ihre Verwendung.

## Beschreibung

Die Erfindung betrifft eine Oxazolinmischung, ein Verfahren zu ihrer Herstellung und ihre Verwendung.

Bekannt sind Verbindungen der Formel VII 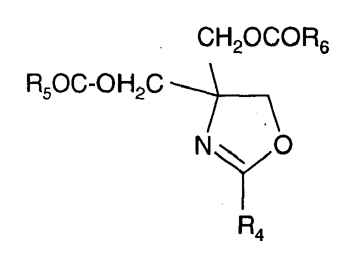 bei denen R₄, R₅ und R₆ gleich sind und einen Alkylrest darstellen, abgeleitet von Propionsäure, Caprylsäure, Capronsäure, Pelargonsäure, Laurinsäure, Myristinsäure, Palmitinsäure, Ölsäure, Stearinsäure, hydrierte Fischölsäure oder Dimerfettsäure. Solche Produkte werden für den Einsatz in Pasten, Fußbodenpflegemitteln, als Zusatzstoffe in Formulierungen der Metallverarbeitung, Kosmetik, Papierverarbeitung und Textilverarbeitung beschrieben (Römpps Chemie Lexikon, 8. Auflage 1985, Band M-Pk, S. 2944 und Anguschemie, TDS 10F Technical Datasheet (http://www.dow.com.angus)).

Diese Verbindungen werden nach dem Stand der Technik in einem einfachen katalysatorfreien Verfahren durch Umsetzung von Trishydroxymethylaminomethan mit Fettsäuren, wie es in Schema I dargestellt ist, hergestellt (Anguschemie, TDS 10F Technical Datasheet (http://www.dow.com.angus)).

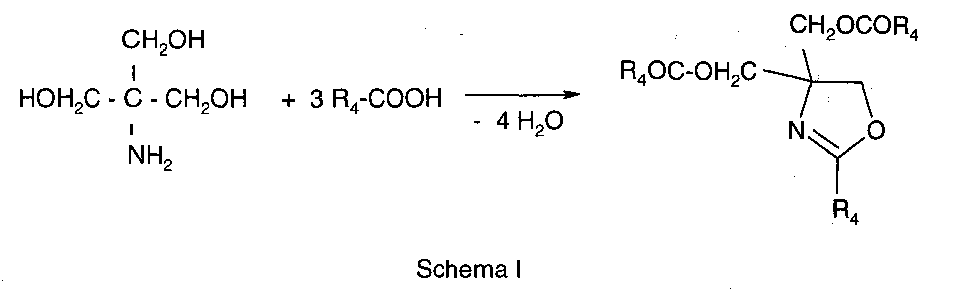

Der Anteil an Fettsäure, R₄-COOH, kann so gewählt werden, dass nicht die gesamte Anzahl an freien Hydroxylgruppen umgesetzt wird.

Die Produkte zeichnen sich durch interessante Eigenschaften aus, so sind sie z.B. in vielen organischen Lösemitteln gut verträglich, zeigen basischen Charakter und sind sowohl am Stickstoff als auch in der Oxazolinalkylkette reaktiv.

Für die Anwendung in Kunststoffen zeigt es sich jedoch, dass solche Verbindungen auf Grund der hohen Dichte an funktionellen Gruppen einen zu polaren Charakter haben. In unpolaren Kunstoffen und Lacksystemen wirken sie zu äußerlich, d.h. sie sind zu unverträglich und reichern sich an der Phasengrenze an und sind dort zu wirksam. In polaren Kunststoffen oder Lacken wirken sie zu innerlich, d.h. sie sind sehr gut verträglich und ihre Wirkung an der Phasengrenze ist zu gering. Dabei bedeutet "äußerlich" eine Anreicherung an der Phasengrenze/Oberfläche und "innerliche Wirkung" eine Wirkung in der Matrix oder eine Wechselwirkung zwischen den Polymermolekülen in der Matrix. Die vorgenannte Phasengrenze bedeutet hier den Übergang von der Schmelze/Feststoffmatrix zu Luft oder zu der Oberfläche des Verarbeitungswerkzeugs.

Durch ihre niedrigen Molgewichte neigen diese Verbindungen außerdem dazu, bei Temperaturbelastung zu migrieren. Das schränkt die Anwendbarkeit dieser Substanzen doch erheblich ein.

Es war daher die Aufgabe gestellt, Produkte mit den charakteristischen Eigenschaften der bekannten Oxazolinderivate der obigen Formel herzustellen, nämlich gute Verträglichkeit mit der organischer Matrix, ausreichende chemische Reaktivität und Basizität, ohne dass deren oben beschriebene Nachteile wie zu hohe Flüchtigkeit und zu starke Migration auftreten.

Gegenstand der Erfindung ist daher eine Oxazolinmischung, dadurch gekennzeichnet, dass sie Oxazoline der Formeln 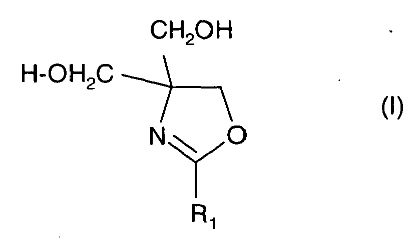 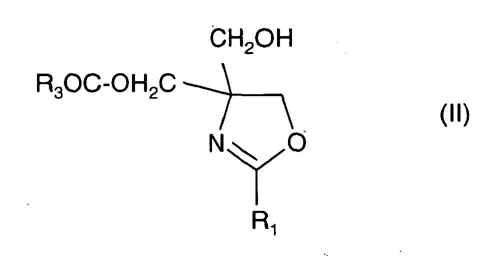 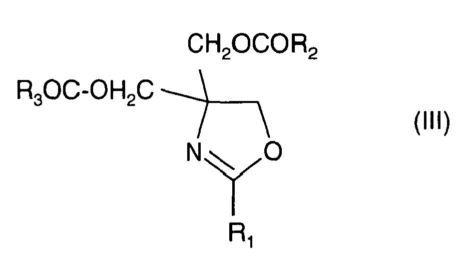 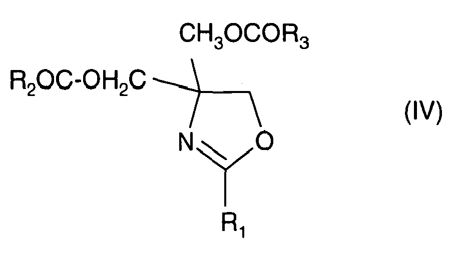 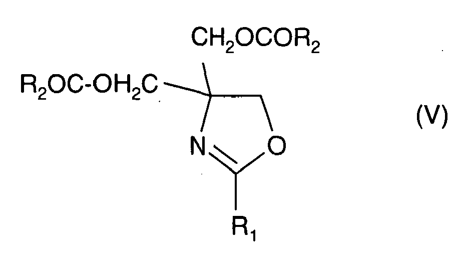 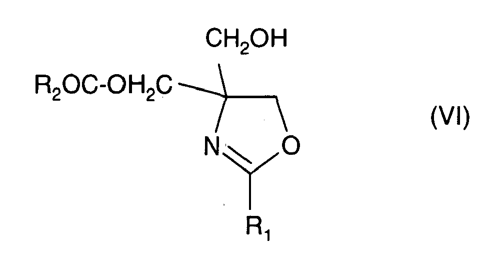 in Mengen von
I = 0 bis 10 Mol-%
II = 1 bis 90 Mol-%
III = 1 bis 98 Mol-%
IV = 1 bis 98 Mol-%
V = 0 bis 50 Mol-%
VI = 0 bis 50 Mol-%
enthält und wobei
R₁ für den Alkylrest einer Fettsäure oder einer Hydroxyfettsäure,
R₂ für den Alkylrest einer Fettsäure, einer Hydroxyfettsäure und/oder der Montanwachssäure und
R₃ für den Alkylrest der Montanwachssäure steht.

Bevorzugt sind in der erfindungsgemäßen Oxazolinmischung die Oxazoline in Mengen von
I = 0 bis 1 Mol-%
II = 2 bis50 Mol-%
III = 10 bis 98 Mol-%
IV = 10 bis 98 Mol-%
V = 0 bis 20 Mol-%
VI = 0 bis 10 Mol-%
enthalten.

Bevorzugt weisen die Alkylreste R₁ und R₂ der Fettsäuren oder der Hydroxyfettsäuren 12 bis 22 C-Atome auf.

Bevorzugt weisen die Alkylreste R₁ und R₂ der Fettsäuren oder der Hydroxyfettsäuren lineare oder verzweigte Alkylketten auf.

Bevorzugt sind die Alkylreste R₁ und R₂ der Fettsäuren oder der Hydroxyfettsäuren gesättigt oder ungesättigt.

Bevorzugt handelt es sich bei dem Alkylrest der Hydroxyfettsäure um den Alkylrest von 12-Hydroxystearinsäure.

Die Erfindung betrifft auch ein Verfahren zur Herstellung einer Oxazolinmischung der Formeln I bis VI, 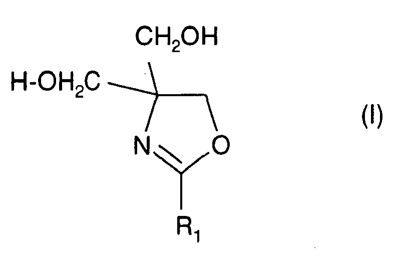 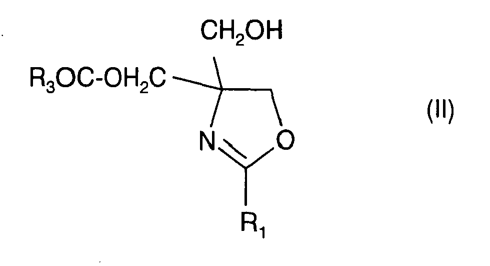 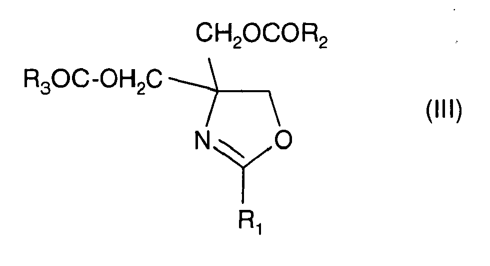 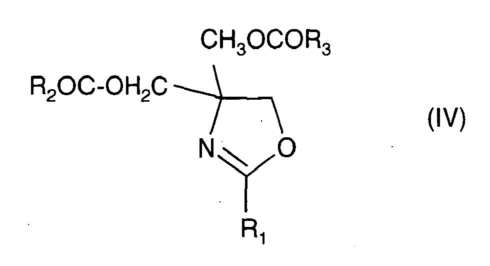 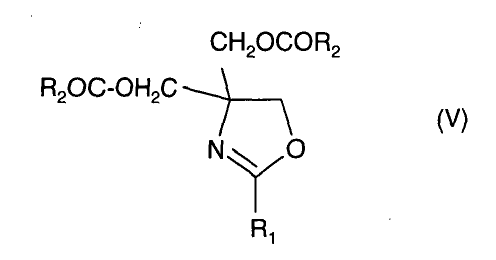 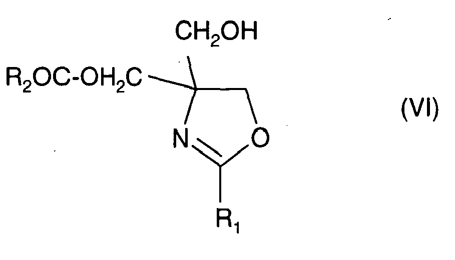 wobei R₁ für den Alkylrest einer Fettsäure oder einer Hydroxyfettsäure, R₂ für den Alkylrest einer Fettsäure, einer Hydroxyfettsäure und/oder der Montanwachssäure und
R₃ für den Alkylrest der Montanwachssäure steht, dadurch gekennzeichnet, dass man Trishydroxymethylaminomethan mit einer Fettsäure und/oder einer Hydroxyfettsäure zu Dihydroxymethyloxazolinen umsetzt und danach die noch freien OH-Gruppen der Dihydroxymethyloxazoline in Gegenwart eines Veresterungskatalysators mit Montanwachssäure verestert werden.

Die Umsetzung läuft dabei nach dem folgenden Verfahrensmuster in drei Schritten ab:

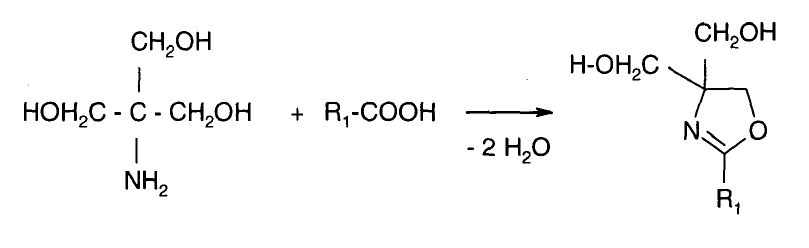

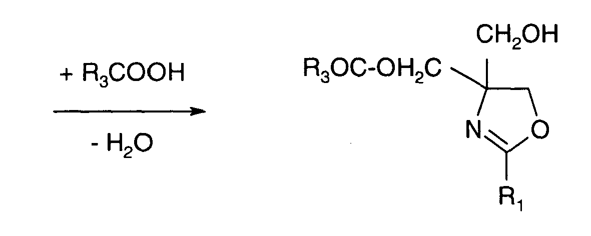

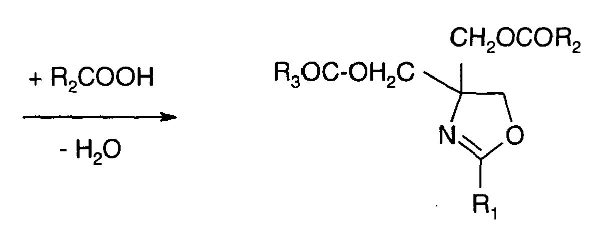

### Schritt 1:

Umsetzung von Trishydroxymethylaminomethan mit einer Fettsäure und/oder einer Hydroxyfettsäure zu Dihydroxymethyloxazolinen (bei dieser Reaktion kann schon ein Teil der freien OH Gruppen auch umgesetzt werden).

### Schritt 2:

Veresterung mit Montanwachssäure R₃-COOH zum Monoester (welcher üblicherweise mindestens 1 mol Montansäure enthält).

### Schritt 3

Veresterung mit einer Carbonsäuremischung, R₂-COOH (wenn R₂ der Alkylrest einer Fettsäure und/oder einer Hydroxyfettsäure ist, beträgt die Umsetzmenge 0 bis 1 mol; wenn R₂ der Alkylrest der Montanwachssäure ist, beträgt die Umsetzmenge 1 bis 2 mol).

Wird bei Schritt 3 die freie OH-Funktion nicht oder nicht vollständig umgesetzt, so entsteht eine Produktmischung aus Oxazolin, Monoester und Diester. Das Mischungsverhältnis der beiden Ester im Produkt kann 1 zu 0 (reiner Monoester) bis 0 zu 1 (reiner Diester) betragen und liegt bevorzugt bei etwa 0,3 zu etwa 0,7 (30 % reiner Monoester und 70 % reiner Diester).

Arbeitet man nicht in drei Schritten, sondern nur in zwei Schritten mit einer Carbonsäuremischung aus R₃-COOH (Montanwachssäure) und R₂-COOH (Fettsäure), so entstehen sowohl die Monoester aus Fettsäure als auch aus Montanwachssäure, und die Diester aus Fettsäure, Fettsäure/Montanwachssäure und Montanwachssäure. Die Verteilung dieser Ester im Produkt richtet sich dann nach der Zusammensetzung der Carbonsäuremischung. Der Anteil an einzusetzender Montanwachssäure sollte deshalb bevorzugt bei 1 bis 2 mol liegen und der Anteil an einzusetzender Fettsäure bei 0 bis 1. Sollen noch Monoester im Produktmix enthalten sein, wird der Anteil der Fettsäure entsprechend reduziert. Die möglichen Produkte sind nachfolgend gezeichnet:

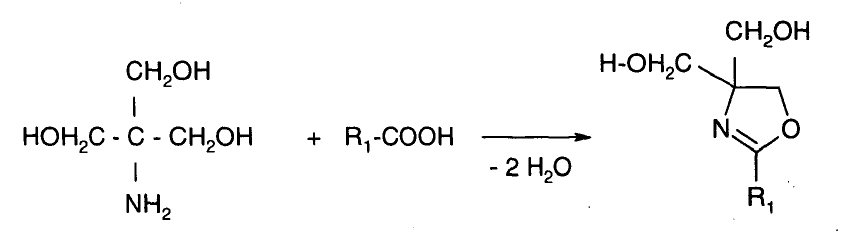

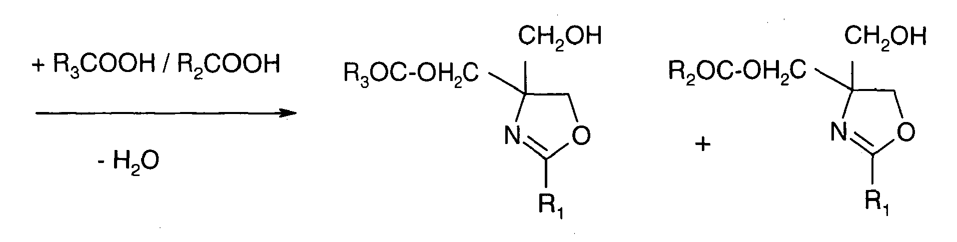

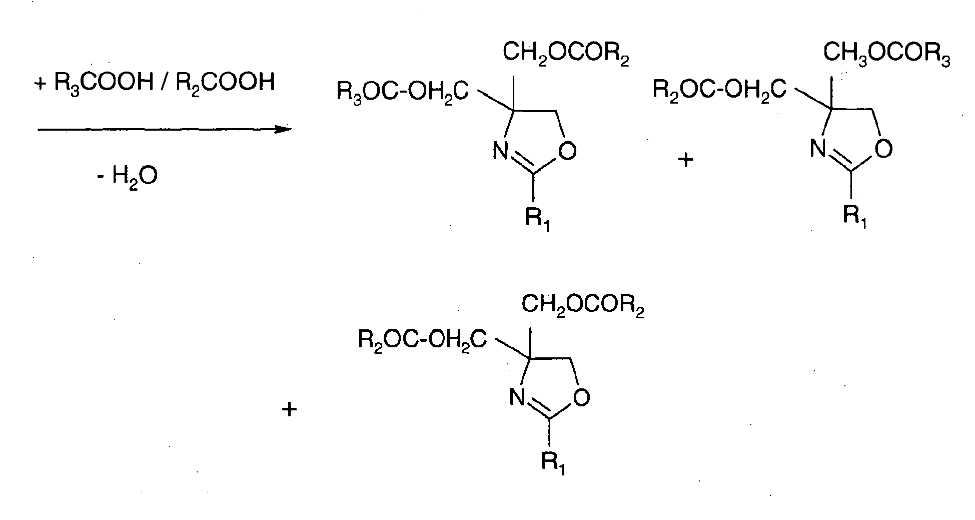

Bevorzugt enthält beim erfindungsgemäßen Verfahren die Oxazolinmischung Oxazoline der Formeln I bis VI in Mengen von
I = 0 bis 10 Mol-%
II = 1 bis 90 Mol-%
III = 1 bis 98 Mol-%
IV = 1 bis 98 Mol-%
V = 0 bis 50 Mol-%
VI = 0 bis 50 Mol-%.

Besonders bevorzugt enthält beim erfindungsgemäßen Verfahren die
Oxazolinmischung Oxazoline der Formeln I bis VI in Mengen von
I = 0 bis 1 Mol-%
II = 2 bis 50 Mol-%
III = 10 bis 98 Mol-%
IV = 10 bis 98 Mol-%
V = 0 bis 20 Mol-%
VI = 0 bis 10 Mol-%.

Bevorzugt ist der Veresterungskatalysator aus der Gruppe der Zinnverbindungen der Gruppe der Titansäureester, Zinkoxide und/oder Zinkseifen ausgewählt.

Bevorzugt weisen die Fettsäuren oder Hydroxyfettsäuren 12 bis 22 C-Atome auf. Bevorzugt weisen die Fettsäuren oder Hydroxyfettsäuren lineare oder verzweigte Alkylketten auf.

Bevorzugt sind die Fettsäuren oder Hydroxyfettsäuren ungesättigt.

Bevorzugt setzt man beim erfindungsgemäßen Verfahren zunächst 1 mol Trishydroxymethylaminomethan mit 1 bis 2,0 mol einer Fettsäure und/oder Hydroxyfettsäure um und dann das entstandene Produkt mit 1,0 bis 1,9 mol Montanwachssäure.

Besonders bevorzugt setzt man dabei zunächst 1 mol Trishydroxymethylaminomethan mit 1,1 bis 1,5 mol einer Fettsäure und/oder Hydroxyfettsäure um und dann das entstandene Produkt mit 1,0 bis 1,9 mol Montanwachssäure.

Die Erfindung betrifft auch die Verwendung der Oxazolinmischung gemäß den Ansprüchen 1 bis 6 als Verabeitungshilfsmittel für Kunststoffe.

Die Erfindung betrifft ebenfalls die Verwendung der Oxazolinmischung gemäß den Ansprüchen 1 bis 6 als Dispergierhilfsmittel für Pigmente und Kunststoffadditive.

Bevorzugt handelt es sich bei den Kunststoffen um Polystyrol-HI (High-Impact), Polyphenylenether, Polyamide, Polyester, Polycarbonate, Polyoxymethylen (POM), Polyurethane und Blends oder Polymerblends vom Typ ABS (Acrylnitril-Butadien-Styrol) oder PC/ABS (Polycarbonat/Acrylnitril-Butadien-Styrol) oder PPE/HIPS (Polyphenylenether/Polystyrol-HI)-Kunststoffe.

Die Erfindung betrifft auch die Verwendung der Oxazolinmischung gemäß den Ansprüchen 1 bis 6 zur Herstellung von beschichteten organischen oder anorganischen Partikeln.

Die Erfindung betrifft ebenso die Verwendung der Oxazolinmischung gemäß den Ansprüchen 1 bis 6 zur Herstellung von beschichteten organischen oder anorganischen Partikeln, wie sie in Kunststoffen, Farben und Lacken eingesetzt werden.

Die Erfindung betrifft auch organische oder anorganische Partikel, gecoated mit der Oxazolinmischung gemäß den Ansprüchen 1 bis 6.

Bevorzugt handelt es sich bei den organischen Partikeln um unlösliche Mittel wie Lichtschutzmittel, UV-Filter, Prozessstabilisatoren, organische Pigmente und Farbstoffe, Fasern, Antioxidantien, Antistatika und organische Flammschutzmittel.

Zu den geeigneten organischen Partikeln gehören auch HALS (hindered-amino-lightstabilisator), Phenole, Phosphite, Benztriazole und Aluminiumphospholane.

Als zu beschichtende organische Pigmente kommen Monoazo-, Disazo-, verlackte Azo-, β-Naphthol-, Naphthol AS-, Benzimidazolon-, Disazokondensations-, Azo-Metallkomplex-Pigmente und polycyclische Pigmente wie zum Beispiel Phthalocyanin-, Chinacridon-, Perylen-, Perinon-, Thioindigo-, Anthanthron-, Anthrachinon-, Flavanthron-, Indanthron-, Isoviolanthron-, Pyranthron-, Dioxazin-, Chinophthalon-, Isoindolinon-, Isoindolin- und Diketopyrrolopyrrol-Pigmente oder Ruße in Betracht.

Als zu beschichtende organische Farbstoffe kommen Säurefarbstoffe, Direktfarbstoffe, Schwefelfarbstoffe und deren Leukoform, Metallkomplexfarbstoffe oder Reaktivfarbstoffe in Betracht, wobei im Falle der Reaktivfarbstoffe auch mit Nukleophilen abreagierte Farbstoffe eingesetzt werden können.

Bevorzugt handelt es sich bei den anorganischen Partikeln um unlösliche Mittel wie Füllstoffe, Fasern, anorganische Pigmente und anorganische Flammschutzmittel.

Zu den geeigneten anorganischen Partikeln gehören auch Kalk, Talkum, Glasfaser, Eisenoxid und Ammoniumpolyphosphate.

Zu beschichtende anorganische Pigmente sind beispielsweise Titandioxide, Zinksulfide, Eisenoxide, Chromoxide, Ultramarin, Nickel- oder Chromantimontitanoxide, Cobaltoxide sowie Bismutvanadate.

Besonders bevorzugt handelt es sich bei den anorganischen Partikeln um Titandioxid.

Die Erfindung betrifft schließlich auch die Verwendung der mit einer Oxazolinmischung gemäß den Ansprüchen 1 bis 6 gecoateten titandioxidhaltigen Partikeln zur Reduzierung der Formaldehydemmsion von Polyoxymethylen-Formmassen.

Grundsätzlich kommen für die Durchführung des beanspruchten Verfahrens auch Alkanolamine der allgemeinen Formel 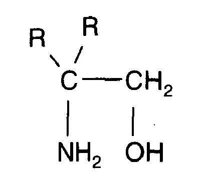 R = H, CH₂-OH, Alkyl
in Frage.

Bevorzugt werden dabei dann 1 mol Trishydroxymethylaminomethan mit 1 bis 2, mol, insbesondere 1,1 bis 1,5 mol einer Fettsäure und dann das entstandene Produkt mit 1,0 bis 1,9 mol, insbesondere 1,5 bis 1,9 mol Montanwachssäure umgesetzt.

Es wurde überraschend gefunden, dass die erfindungsgemäßen Produkte herzustellen sind, wenn nach einem besonders modifizierten Verfahren in einer zweistufigen Verfahrensweise mit Fettsäuren die Stufe der Dihydroxymethyloxazoline hergestellt wird und ohne Aufarbeitung dieser Zwischenstufe die freien OH-Gruppen in Gegenwart eines Katalysators mit Montanwachssäure verestert werden. Solche Produkte haben immer noch eine helle Farbe, sind chemisch reaktiv am Stickstoff und in der Oxazolin-Alkylkette, zeigen eine basische Reaktion, sind gut mit polaren und unpolaren Kunststoffen verträglich aber wesentlich weniger flüchtig als reine Fettsäureprodukte nach dem Stand der Technik und nicht migrierend.

Nach dem Stand der Technik werden die Substanzen gemäß Formel VII durch Umsetzung von Trishydroxymethylaminomethan mit den gewählten Carbonsäuren bei 220 bis 250°C ohne Katalysator hergestellt. Beim Einsatz niedermolekularer Carbonsäuren muss die Reaktion in einem Druckreaktor durchgeführt werden. Die hohen Temperaturen sind erforderlich, um eine ausreichende Umsetzung zu erreichen. Bei den langkettigen Carbonsäuren mit 14 bis 22 C-Atomen wird mit reduziertem Druck gearbeitet. Trotz Verwendung einer Inertatmosphäre kommt es dabei zur Farbvertiefung

### Beispiel 1 (Vergleich)

| Rohstoffe | Menge (g) | mol |
|---|---|---|
| techn. Stearinsäure | 810 | 3 |
| Trishydroxymethylaminomethan | 121 | 1 |

### Beispiel 2 (Vergleich)

| Rohstoffe | Menge (g) | mol |
|---|---|---|
| Tallölfettsäure | 840 | 3 |
| Trishydroxymethylaminomethan | 121 | 1 |

### Kenndaten der Vergleichsprodukte:

| Kenndaten | Tropfpunkt °C | Säurezahl mg KOH | Alkalizahl mg KOH | Farbe | Flüchtigkeit bei 300°C |
|---|---|---|---|---|---|
| Beispiel 1 (Vergleich) | 65 | 12 | 1 | gelblich | 18,0 % |
| Beispiel 2 (Vergleich) | flüssig | 12 | 1 | bräunlich | 23,0 % |

Alle Bestandteile werden in den Reaktor gegeben, dann wird aufgeheizt. Bis zum Erreichen von 165°C wird Stickstoff aufgeleitet. Im Bereich 140 - 165°C muss Schäumen aus Sicherheitsgründen verhindert werden. Bis zum Erreichen der Reaktionstemperatur von 230°C wird mit positivem Druck gearbeitet. Dann wird das Reaktionswasser ausgeschleust, bis die gewünschte Säurezahl erreicht ist.

### Beispiele nach erfindungsgemäßem Verfahren (Beispiele 3 bis 6):

Die jeweilige Fettsäure bzw. Hydroxyfettsäure und das Trishydroxymethylaminomethan werden in den Reaktor gegeben, es wird Vakuum angelegt und mit Stickstoff belüftet, dann wird erhitzt und die Fettsäure geschmolzen. In die Schmelze dosiert man geringe Mengen Dispergierhilfsmittel und heizt 40 bis 80°C oberhalb des Schmelzpunktes der Fettsäure auf. Bei dieser Temperatur wird gehalten, bis 2 mol Wasser pro mol eingesetztem Amin entstanden sind.

In die Schmelze wird dann die flüssige Montanwachssäure eindosiert, es wird noch Veresterungskatalysator zugesetzt und gerührt, bis die Säurezahl auf <15 abgesunken ist. Der Ansatz wird dann gekühlt und granuliert.

### Beispiel 3

| Rohstoffe | Menge (g) | mol |
|---|---|---|
| Trishydroxymethylaminomethan | 121 | 1 |
| techn. Stearinsäure | 300 | 1,1 |
| techn. Montanwachssäure | 750 | 1,6 |
| Veresterungskatalysator | 1,2 | |

### Beispiel 4

| Rohstoffe | Menge (g) | mol |
|---|---|---|
| Trishydroxymethylaminomethan | 121 | 1 |
| techn. 12-Hydroxystearinsäure | 330 | 1,1 |
| techn. Montanwachssäure | 750 | 1,6 |
| Veresterungskatalysator | 1,2 | |
| Dispergierhilfsmittel | 1,2 | |

### Beispiel 5

| Rohstoffe | Menge (g) | mol |
|---|---|---|
| Trishydroxymethylaminomethan | 121 | 1 |
| techn. 12-Hydroxystearinsäure | 600 | 2 |
| techn. Montanwachssäure | 375 | 0,8 |
| Veresterungskatalysator | 1,2 | |

### Beispiel 6

| Rohstoffe | Menge (g) | mol |
|---|---|---|
| Trishydroxymethylaminomethan | 121 | 1 |
| techn. 12-Hydroxystearinsäure | 900 | 3 |
| Veresterungskatalysator | 1,2 | |

### Ergebnisse

| Kenndaten | Tropfpunkt °C | Säurezahl mg KOH | Alkalizahl mg KOH | Farbe | Flüchtigkeit bei 300°C |
|---|---|---|---|---|---|
| Beispiel 3 | 74 | 15 | 0 | gelblich | 6,0 % |
| Beispiel 4 | 72 | 13 | 0 | gelblich | 5,5 % |
| Beispiel 5 | 70 | 12 | 0 | hellgelb | 9,0 % |
| Beispiel 6 | 65 | 12 | 0 | weiß | 15,0 % |

### Meßmethoden:

Tropfpunkt: nach DIN 51801/2
Säurezahl: nach DIN 53402
Alkalizahl: nach DGF - M IV-4 (63)
Flüchtigkeit: nach interner Methode über thermogravimetrische Analyse (TGA):

| | |
|---|---|
| Temperaturbereich: | 30 - 300°C |
| Aufheizrate: | 2 K/min |
| Isotherm: | 120 min bei 300°C |
| Stickstoffspülung | 50 ml/min |
| Einwaage: | 50 mg +/-0,3 mg |
| Aluminiumoxidtiegel: | 150 µl |

®Licowachs C ist ein Handelsprodukt der Clariant GmbH auf Basis von Ethylendiamin und Talgfettsäure.

### Anwendungsbeispiel 1

Technisches Polyurethan (TPU) wird mit 0,2 % Gleitmittel ausgerüstet und standardmäßig verarbeitet. Geprüft wurden die Gleitmittelwirkung und die Migrationsneigung bei Wärmelagerung. Bewertet werden die Eigenschaften nach Schulnoten, d.h. je niedriger die Note, desto besser die Gleitwirkung.

Verarbeitung von TPU mit ®Licowachs C und Produkt nach Beispiel 5

| | ®Licowachs C | Oxazolin nach Beispiel 5 |
|---|---|---|
| Gleitmittelwirkung | 2 | 2 |
| Migrationsstabilität bei 40°C | 4 | 2 |
| Migrationsstabilität bei 60°C | 4-5 | 2 |

Erfindungsgemäße Umsetzungsprodukte aus Trishydroxymethylaminomethan, einer Fettsäure und Montanwachssäure zeigen gute Verträglichkeit mit Kunststoffen, geringe Migrationsneigung und geringe Flüchtigkeit. Dies bewirkt eine verbesserte Performance gegenüber Produkten des Standes der Technik. Das zeigt sich u.a. darin, dass eine ausgewogene Verteilung von innerer und äußerer Wirkung zu beobachten ist, d.h. Gleitmittelwirkung nach außen und dispergierende Wirkung und chemische Wirkung im Inneren.

### Anwendungsbeispiel 2

Titandioxid der Type R 104 (DuPont) wurde in einem Heißmischer bei 90°C mit dem erfindungsgemäßen Produkt aus Beispiel 4 gecoated. Der Anteil des Produktes lag bei 2 und 4 Gew.-%. Die so modifizierten Pigmente wurden in POM eingearbeitet und auf Freisetzung von Formaldehyd geprüft. Verglichen wird mit Standardprodukt und einem kommerziellen Gleitmittel auf Fettbasis.

### Formaldehydemission nach VDA275

Aus den POM-Formmassen werden Platten der Wandstärke 1 mm gefertigt.
Nach einer Lagerdauer von 24 h wurde die Formaldehydemission aus den Platten nach VDA 275 ermittelt (VDA Empfehlung Nr. 275, Dokumentation Kraftfahrwesen e.V. Juli 1994).

Prüfkörperherstellung: Das Polyacetalgranulat wurde durch Spritzguss zu Plättchen mit den Dimensionen 80*50*1 mm geformt. Eine Spritzgießmaschine Kraus Maffei KM 120/340B wurde mit folgenden Spritzgießparametern verwendet:
Massetemperatur 195°C, Fließfrontgeschwindigkeit 200 mm/s, Werkzeugwandtemperatur 85°C, Nachdruck 900 bar, Nachdruckzeit 30 s, Kühlzeit 10 s, Staudruck 0 bis 10 bar.

Die Prüfkörper wurden vor der Prüfung für 24 h im Norm-Klimaschrank bei 23°C und 50 relativer Luftfeuchte gelagert.
Prüfung: Zwei Prüfkörper wurden in einer 1 I Glasflasche über 50 ml E-Wasser an einem Edelstahlhacken aufgehängt und für 3 h im Umlufttrockenschrank bei 60°C gelagert. Die Prüfkörper wurden aus der Prüfflasche entfernt. 5 ml Probenlösung wurden in ein Reagenzglas pipettiert, das Reagenzglas wurde für 10 Minuten bei 95°C getempert. Nun wurden 3 ml Acetylaceton und 3 ml einer 20 %igen Ammoniumacetatlösung in das Reagenzglas hinzugegeben. Der Formaldehyd bildete mit den Reagenzien den Diacetyldihydrolutidin-Komplex, dessen Absorption bei 412 nm photometrisch bestimmt wurde. Aus der Absorption wurde die Formaldehydkonzentration in der Probenlösung berechnet.

Verarbeitung von pigmentiertem POM (Polyoxymethylen)

| Polymer | Gleitmittel | Menge mol-% | VDA 275 ppm/h |
|---|---|---|---|
| Copolymer 2 | ®Licowachs C | 0,20 | 118 |
| Copolymer 2 | Pigment + 4 % Produkt aus Beispiel 4 | 0,5 | 32 |
| Copolymer 2 | Pigment + 2 % Produkt aus Beispiel 4 | 0,2 | 37 |

| | | | |
|---|---|---|---|
| Copolymer 2: ®Hostaform 27021 der Ticona GmbH | | | |
| ®Licowachs C: Ethylenbisstearamid, Handelsprodukt der Clariant GmbH. | | | |

Der Einsatz von mit dem erfindungsgemäßen Produkt modifiziertem Pigment zeigt deutlich geringere Formaldehydemissionen im Vergleich zum Einsatz von unmodifiziertem Pigment in Kombination mit Standardgleitmittel auf Fettbasis (®Licowachs C).

## Patentansprüche

1. Oxazolinmischung, **dadurch gekennzeichnet, dass** sie Oxazoline der Formeln 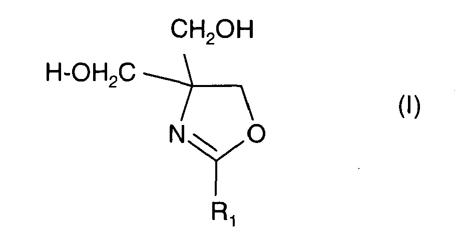 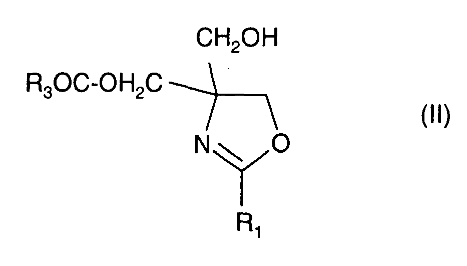 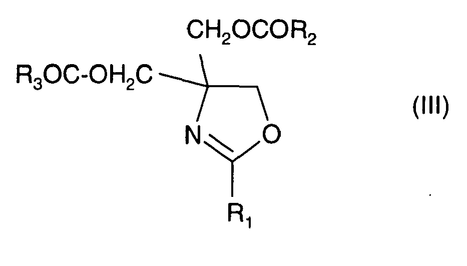 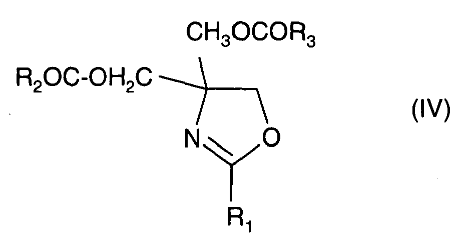 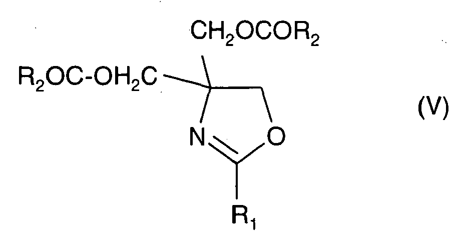 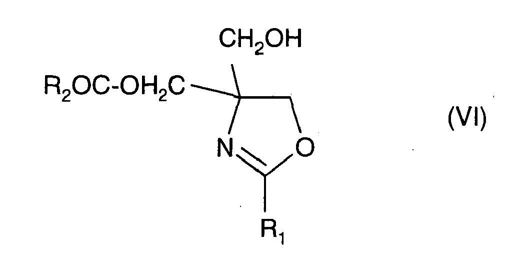 in Mengen von
I=0 bis 10 Mol-%
II = 1 bis 90 Mol-%
III = 1 bis 98 Mol-%
IV = 1 bis 98 Mol-%
V = 0 bis 50 Mol-%
VI = 0 bis 50 Mol-%
enthält, wobei
R₁ für den Alkylrest einer Fettsäure oder einer Hydroxyfettsäure,
R₂ für den Alkylrest einer Fettsäure, einer Hydroxyfettsäure und/oder der Montanwachssäure und
R₃ für den Alkylrest der Montanwachssäure steht.

2. Oxazolinmischung nach Anspruch 1, **dadurch gekennzeichnet, dass** die Oxazoline in Mengen von
I = 0 bis 1 Mol-%
II = 2 bis 50 Mol-%
III = 10 bis 98 Mol-%
IV = 10 bis 98 Mol-%
V = 0 bis 20 Mol-%
VI = 0 bis 10 Mol-%
enthalten sind.

3. Oxazolinmischung nach Anspruch 1 oder 2, **dadurch gekennzeichnet, dass** die die Alkylreste R₁ und R₂ der Fettsäuren oder der Hydroxyfettsäuren 12 bis 22 C-Atome aufweisen.

4. Oxazolinmischung nach einem oder mehreren der Ansprüche 1 bis 3, **dadurch gekennzeichnet, dass** die die Alkylreste R₁ und R₂ der Fettsäuren oder der Hydroxyfettsäuren lineare oder verzweigte Alkylketten aufweisen.

5. Oxazolinmischung nach einem oder mehreren der Ansprüche 1 bis 4, **dadurch gekennzeichnet, dass** die die Alkylreste R₁ und R₂ der Fettsäuren oder der Hydroxyfettsäuren gesättigt oder ungesättigt sind.

6. Oxazolinmischung nach einem oder mehreren der Ansprüche 1 bis 5, **dadurch gekennzeichnet, dass** es sich bei der Hydroxyfettsäure um 12 Hydroxystearinsäure handelt.

7. Verfahren zur Herstellung einer Oxazolinmischung der Formeln I bis VI, 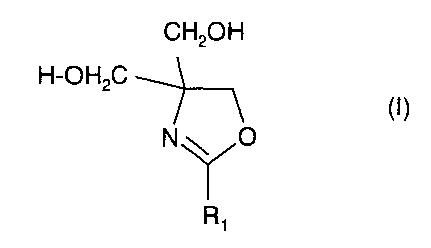 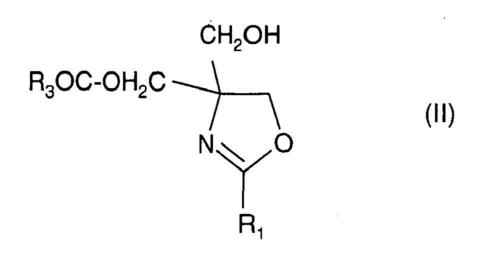 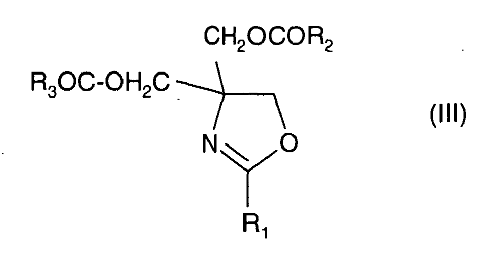 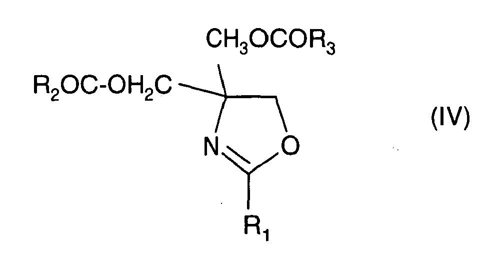 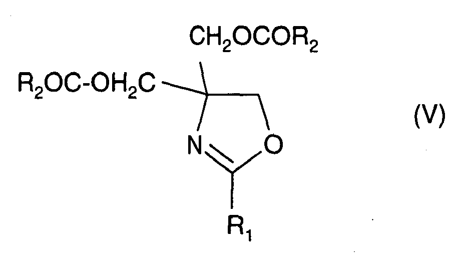 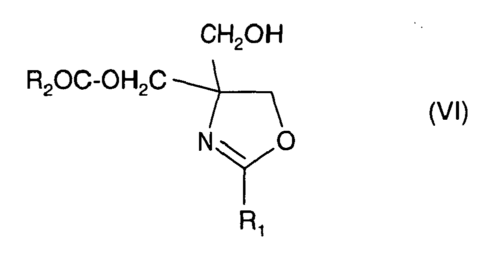 wobei R₁ für den Alkylrest einer Fettsäure oder einer Hydroxyfettsäure, R₂ für den Alkylrest einer Fettsäure, einer Hydroxyfettsäure und/oder der Montanwachssäure und
R₃ für den Alkylrest der Montanwachssäure steht, **dadurch gekennzeichnet, dass** man Trishydroxymethylaminomethan mit einer Fettsäure und/oder einer Hydroxyfettsäure zu Dihydroxymethyloxazolinen umsetzt und danach die noch freien OH-Gruppen der Dihydroxymethyloxazoline in Gegenwart eines Veresterungskatalysators mit Montanwachssäure verestert werden.

8. Verfahren nach Anspruch 7, **dadurch gekennzeichnet, dass** die Oxazolinmischung Oxazoline der Formeln I bis VI in Mengen von
I = 0 bis 10 Mol-%
II = 1 bis 90 Mol-%
III = 1 bis 98 Mol-%
IV = 1 bis 98 Mol-%
V = 0 bis 50 Mol-%
VI = 0 bis 50 Mol-%
enthält.

9. Verfahren nach Anspruch 7 oder 8, **dadurch gekennzeichnet, dass** die Oxazolinmischung Oxazoline der Formeln 1 bis VI in Mengen von
I = 0 bis 1 Mol-%
II = 2 bis 50 Mol-%
III = 10 bis 98 Mol-%
IV = 10 bis 98 Mol-%
V = 0 bis 20 Mol-%
VI = 0 bis 10 Mol-%
enthält.

10. Verfahren nach Anspruch 9 , **dadurch gekennzeichnet, dass** der Veresterungskatalysator aus der Gruppe der Zinnverbindungen, der Gruppe der Titansäureester, Zinkoxide und/oder Zinkseifen ausgewählt ist.

11. Verfahren nach Anspruch 9 oder 10, **dadurch gekennzeichnet, dass** die Fettsäuren oder Hydroxyfettsäuren 12 bis 22 C-Atome aufweisen.

12. Verfahren nach einem oder mehreren der Ansprüche 9 bis 11, **dadurch gekennzeichnet, dass** die Fettsäuren oder Hydroxyfettsäuren lineare oder verzweigte Alkylketten aufweisen.

13. Verfahren nach einem oder mehreren der Ansprüche 9 bis 12, **dadurch gekennzeichnet, dass** die Fettsäuren oder Hydroxyfettsäuren gesättigt oder ungesättigt sind.

14. Verfahren nach einem oder mehreren der Ansprüche 9 bis 13, **dadurch gekennzeichnet, dass** die Alkylketten funktionelle Gruppen aufweisen.

15. Verfahren nach einem oder mehreren der Ansprüche 9 bis 14, **dadurch gekennzeichnet, dass** man zunächst 1 mol Trishydroxymethylaminomethan mit 1 bis 2,0 mol einer Fettsäure und dann das entstandene Produkt mit 1,0 bis 1,9 mol Montanwachssäure umsetzt.

16. Verfahren nach einem oder mehreren der Ansprüche 9 bis 15, **dadurch gekennzeichnet, dass** man zunächst 1 mol Trishydroxymethylaminomethan mit 1,1 bis 1,5 mol einer Fettsäure und dann das entstandene Produkt mit 1,0 bis 1,9 mol Montanwachssäure umsetzt.

17. Verwendung einer Oxazolinmischung nach mindestens einem der Ansprüche 1 bis 6 als Verarbeitungshilfsmittel für Kunststoffe.

18. Verwendung einer Oxazolinmischung nach mindestens einem der Ansprüche 1 bis 6 als Dispergierhilfsmittel für Pigmente und Kunststoffadditive.

19. Verwendung nach Anspruch 17 oder 18, **dadurch gekennzeichnet, dass** es sich bei den Kunststoffen um Polystyrol-HI (High-Impact), Polyphenylenether, Polyamide, Polyester, Polycarbonate, Polyoxymethylen (POM), Polyurethane und Blends oder Polymerblends vom Typ ABS (Acrylnitril-Butadien-Styrol) oder PC/ABS (Polycarbonat/ Acrylnitril-Butadien-Styrol) oder PPE/HIPS (Polyphenylenether/Polystyrol-HI)-Kunststoffe handelt.

20. Verwendung einer Oxazolinmischung nach mindestens einem der Ansprüche 1 bis 6 zur Herstellung von beschichteten organischen oder anorganischen Partikeln.

21. Verwendung einer Oxazolinmischung nach mindestens einem der Ansprüche 1 bis 6 zur Herstellung von beschichteten organischen oder anorganischen Partikeln, wie sie in Kunststoffen, Farben und Lacken eingesetzt werden.

22. Verwendung nach Anspruch 20 oder 21, **dadurch gekennzeichnet, dass** es sich bei den organischen Partikeln um unlösliche Mittel handelt wie Lichtschutzmittel, UV-Filter, Prozessstabilisatoren, organische Pigmente, Fasern, Antioxidantien, Antistatika und organische Flammschutzmittel.

23. Verwendung nach Anspruch 20 oder 21, **dadurch gekennzeichnet, dass** es sich bei den anorganischen Partikeln um unlösliche Mittel handelt wie Füllstoffe, Fasern, anorganische Pigmente und anorganische Flammschutzmittel.

24. Organische oder anorganische Partikel, gecoated mit einer Oxazolinmischung nach mindestens einem der Ansprüche 1 bis 6.

25. Partikel nach Anspruch 24, **dadurch gekennzeichnet, dass** es sich bei den organischen Partikeln um unlösliche Mittel wie Lichtschutzmittel, UV-Filter, Prozessstabilisatoren, organische Pigmente, Fasern, Antioxidantien, Antistatika und organische Flammschutzmittel handelt.

26. Partikel nach Anspruch 24, **dadurch gekennzeichnet, dass** es sich bei den anorganischen Partikeln um unlösliche Mittel wie Füllstoffe, Fasern, anorganische Pigmente und anorganische Flammschutzmittel handelt.

27. Partikel nach Anspruch 26, **dadurch gekennzeichnet, dass** es sich bei den anorganischen Partikeln um Titandioxid handelt.

28. Verwendung der mit einer Oxazolinmischung gecoateten anorganischen titandioxidhaltigen Partikel nach Anspruch 27 zur Reduzierung der Formaldehydemmsion von Polyoxymethylen-Formmassen.
